# EUROPÄISCHE PATENTSCHRIFT

(11) **EP 1 603 895 B1**
(45) Veröffentlichungstag und Bekanntmachung des Hinweises auf die Patenterteilung: **20.02.2008**
(21) Anmeldenummer: 04713467.1
(22) Anmeldetag: 21.02.2004
(51) Int. Cl.: C07D 401/04, A61K 31/444

(54) **SUBSTITUIERTE BENZOYLUREIDOPYRIDYL-PIPERIDIN- UND -PYRROLIDIN-CARBONS UREDERIVATE, VERFAHREN ZU DEREN HERSTELLUNG UND DEREN V ERWENDUNG**
SUBSTITUTED BENZOYLUREIDOPYRIDYL-PIPERIDINE AND -PYRROLIDINE CARBOXYLIC ACID DERIVATIVES, METHOD FOR THE PRODUCTION THEREOF AND THE USE THEREOF
DERIVES SUBSTITUES DE L'ACIDE BENZOYLUREIDOPYRIDYL-PIPERIDINE-CARBOXYLIQUE ET DE L'ACIDE BENZOYLUREIDOPYRIDYL-PYRROLIDINE-CARBOXYLIQUE, LEUR PROCEDE DE PRODUCTION ET LEUR UTILISATION

(30) Priorität: 07.03.2003 DE 10309929
(43) Veröffentlichungstag der Anmeldung: 14.12.2005
(73) Patentinhaber: Sanofi-Aventis Deutschland GmbH, 65929 Frankfurt am Main (DE)
(72) Erfinder: SCHOENAFINGER, Karl, 63755 Alzenau (DE); KADEREIT, Dieter, 65926 Frankfurt am Main (DE); DEFOSSA, Elisabeth, 65510 Idstein (DE); HERLING, Andreas, 65520 Bad Camberg (DE); KLABUNDE, Thomas, 65929 Frankfurt (DE)
(86) Internationale Anmeldenummer: PCT/EP2004/001735
(87) Internationale Veröffentlichungsnummer: WO 2004/078743

(56) Entgegenhaltungen:
- WO-A-01/94300
- WO-A-02/096864
- WO-A-03/084922
- WO-A-20/04007437
- WO-A-20/04007455

## Beschreibung

Die Erfindung betrifft substituierte Benzoylureidopyridyl-piperidin- und -pyrrolidincarbonsäurederivate sowie deren physiologisch verträgliche Salze.

Der Erfindung lag die Aufgabe zugrunde, Verbindungen zur Verfügung zu stellen, mit denen eine Prävention und Behandlung von Diabetes Mellitus möglich ist. Die Verbindungen sollten dazu insbesondere eine therapeutisch verwertbare Blutzucker senkende Wirkung entfalten.

Die Erfindung betrifft Verbindungen der Formel I, 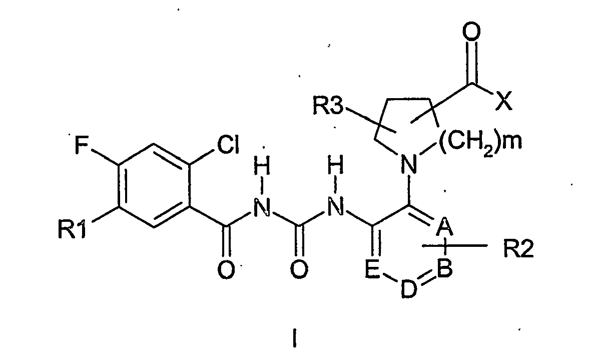 worin bedeuten
- R1, R2: unabhängig voneinander H, F, Cl, Br, (C₁-C₆)-Alkyl, CF₃, OCF₃, NO₂, CN, O-(C₁-C₆)-Alkyl, COO(C₁-C₆)-Alkyl, COOH, CO-(C₁-C₆)-Alkyl, (C₀-C₆)-Alkyl-COOH, (C₀-C₆)-Alkyl -COO(C₁-C₆)-Alkyl, SO₂-(C₁-C₆)-Alkyl;
- R3: H, (C₁-C₆)-Alkyl, (C₀-C₆)-Alkyl-Aryl, O-(C₁-C₆)-Alkyl, O-(C₂-C₆)-Alkenyl, O-(C₂-C₆)-Alkinyl, wobei die Alkyl-, Aryl-, Alkenyl- und Alkinylreste ein oder mehrfach durch F, Cl oder Br substituiert sein können;
- X: OH, O-(C₁-C₆)-Alkyl, NH₂, NH(C₁-C₆)-Alkyl, N((C₁-C₆)-Alkyl)₂;
- A, B, D, E: unabhängig voneinander CH oder N, wobei mindestens eine der Gruppen A, B, D und E die Bedeutung N hat;
- m: 0, 1, 2;
sowie deren physiologisch verträgliche Salze.

Bevorzugt sind Verbindungen der Formel I worin ein-oder mehrere Reste folgende Bedeutung haben:
- R1, R2: unabhängig voneinander H, F, Cl, Br, (C₁-C₆)-Alkyl, CF₃, OCF₃, NO₂, CN, O-(C₁-C₆)-Alkyl, COO(C₁-C₆)-Alkyl, COOH, CO-(C₁-C₆)-Alkyl, (C₀-C₆)-Alkyl-COOH, (C₀-C₆)-Alkyl-COO(C₁-C₆)-Alkyl, SO₂-(C₁-C₆)-Alkyl;
- R3: H, (C₁-C₆)-Alkyl, (C₀-C₆)-Alkyl-Aryl, O-(C₁-C₆)-Alkyl, O-(C₂-C₆)-Alkenyl, O-(C₂-C₆)-Alkinyl, wobei die Alkyl-, Aryl-, Alkenyl- und Alkinylreste ein oder mehrfach durch F, Cl oder Br substituiert sein können;
- X: OH, O-(C₁-C₆)-Alkyl, NH₂, NH(C₁-C₆)-Alkyl, N((C₁-C₆)-Alkyl)₂;
- A, B, D, E: unabhängig voneinander CH oder N, wobei mindestens eine der Gruppen A, B, D und E die Bedeutung N hat;
- m: 1, 2;
sowie deren physiologisch verträgliche Salze.

Besonders bevorzugt sind Verbindungen der Formel I worin ein oder mehrere Reste folgende Bedeutung haben:
- R1: H, F;
- R2: unabhängig voneinander H, F, Cl, Br, (C₁-C₆)-Alkyl, CF₃, OCF₃, O-(C₁-C₆)-Alkyl, COO(C₁-C₆)-Alkyl, COOH, CO-(C₁-C₆)-Alkyl, (C₀-C₆)-Alkyl-COOH, (C₀-C₆)-Alkyl-COO(C₁-C₆)-Alkyl, SO₂-(C₁-C₆)-Alkyl;
- R3: H, (C₁-C₆)-Alkyl, (C₀-C₆)-Alkyl-Aryl, O-(C₁-C₆)-Alkyl, O-(C₂-C₆)-Alkenyl, O-(C₂-C₆)-Alkinyl, wobei die Alkyl-, Aryl-, Alkenyl- und Alkinylreste ein oder mehrfach durch F, Cl oder Br substituiert sein können;
- X: OH, O-(C₁-C₆)-Alkyl, NH₂, NH(C₁-C₆)-Alkyl, N((C₁-C₆)-Alkyl)₂;
- A: N
- B, D, E: CH
- m: 1, 2;
sowie deren physiologisch verträgliche Salze.

Ganz besonders bevorzugt sind Verbindungen der Formel worin ein oder mehrere Reste folgende Bedeutung haben:
- R1: H, F;
- R2: H, Cl, (C₁-C₆)-Alkyl, CF₃, COO(C₁-C₆)-Alkyl, COOH,
- R3: H, Phenyl;
- X: OH, O-(C₁-C₆)-Alkyl, NH₂, NH(C₁-C₆)-Alkyl, N((C₁-C₆)-Alkyl)₂;
- A: N;
- B, D, E: CH;
- m: 2;
sowie deren physiologisch verträgliche Salze.

Die Erfindung bezieht sich auf Verbindungen der Formel I, in Form ihrer Razemate, razemischen Mischungen und reinen Enantiomeren sowie auf ihre Diastereomeren und Mischungen davon.

Die Alkylreste in den Substituenten R1, R2, und R3, können sowohl geradkettig wie verzweigt sein.

Können Reste oder Substituenten mehrfach in den Verbindungen der Formel I auftreten, so können sie alle unabhängig voneinander die angegebenen Bedeutungen haben und gleich oder verschieden sein.

Pharmazeutisch verträgliche Salze sind aufgrund ihrer höheren Wasserlöslichkeit gegenüber den Ausgangs- bzw. Basisverbindungen besonders geeignet für medizinische Anwendungen. Diese Salze müssen ein pharmazeutisch verträgliches Anion oder Kation aufweisen. Geeignete pharmazeutisch verträgliche Säureadditionssalze der erfindungsgemäßen Verbindungen sind Salze anorganischer Säuren, wie Salzsäure, Bromwasserstoff-, Phosphor-, Metaphosphor-, Salpeter- und Schwefelsäure sowie organischer Säuren, wie z.B. Essigsäure, Benzolsulfon-, Benzoe-, Zitronen-, Ethansulfon-, Fumar-, Glucon-, Glykol-, Isethiön-, Milch-, Lactobion-, Malein-, Äpfel-, Methansulfon-, Bernstein-, p-Toluolsulfon- und Weinsäure.

Geeignete pharmazeutisch verträgliche basische Salze sind Ammoniumsalze, Alkalimetallsalze (wie Natrium- und Kaliumsalze), Erdalkalisalze (wie Magnesium- und Calciumsalze), Trometamol (2-Amino-2-hydroxymethyl-1,3-propandiol), Diethanolamin, Lysin, oder Ethylendiamin.

Salze mit einem nicht pharmazeutisch verträglichen Anion, wie zum Beispiel Trifluoracetat, gehören ebenfalls in den Rahmen der Erfindung als nützliche Zwischenprodukte für die Herstellung oder Reinigung pharmazeutisch verträglicher Salze und/oder für die Verwendung in nicht-therapeutischen, zum Beispiel in-vitro-Anwendungen.

Nachfolgend beziehen sich alle Verweise auf "Verbindung(en) gemäß Formel I" auf Verbindung(en) der Formel I wie vorstehend beschrieben, sowie ihre Salze.

Unter einem Arylrest wird ein Phenyl-, Naphthyl-, Biphenyl-, Tetrahydronaphthyl-, alpha- oder beta-Tetralon-, Indanyl- oder Indan-1-on-ylrest verstanden.

Die Verbindung(en) der Formel (I) können auch in Kombination mit weiteren Wirkstoff verabreicht werden.

Die Menge einer Verbindung gemäß Formel I, die erForderlich ist, um den gewünschten biologischen Effekt zu erreichen, ist abhängig von einer Reihe von Faktoren, z.B. der gewählten spezifischen Verbindung, der beabsichtigten Verwendung, der Art der Verabreichung und dem klinischen Zustand des Patienten. Im allgemeinen liegt die Tagesdosis im Bereich von 0,3 mg bis 100 mg (typischerweise von 3 mg und 50 mg) pro Tag pro Kilogramm Körpergewicht, z.B. 3-10 mg/kg/Tag. Eine intravenöse Dosis kann z.B. im Bereich von 0,3 mg bis 1,0 mg/kg liegen, die in geeigneter Weise als Infusion von 10 ng bis 100 ng pro Kilogramm pro Minute verabreicht werden kann. Geeignete Infusionslösungen für diese Zwecke können z.B. von 0,1 ng bis 10 mg, typischerweise von 1 ng bis 10 mg pro Milliliter, enthalten. Einzeldosen können z.B. von 1 mg bis 10 g des Wirkstoffs enthalten. Somit können Ampullen für Injektionen beispielsweise von 1 mg bis 100 mg, und oral verabreichbare Einzeldosisformulierungen, wie zum Beispiel Tabletten oder Kapseln, können beispielsweise von 1,0 bis 1000 mg, typischerweise von 10 bis 600 mg enthalten. Zur Therapie der oben genannten Zustände können die Verbindungen gemäß Formel I selbst als Verbindung verwendet werden, vorzugsweise liegen sie jedoch mit einem verträglichen Träger in Form einer pharmazeutischen Zusammensetzung vor. Der Träger muss natürlich verträglich sein, in dem Sinne, dass er mit den anderen Bestandteilen der Zusammensetzung kompatibel ist und nicht gesundheitsschädlich für den Patienten ist. Der Träger kann ein Feststoff oder eine Flüssigkeit oder beides sein und wird vorzugsweise mit der Verbindung als Einzeldosis formuliert, beispielsweise als Tablette, die von 0,05% bis 95 Gew.-% des Wirkstoffs enthalten kann. Weitere pharmazeutisch aktive Substanzen können ebenfalls vorhanden sein, einschließlich weiterer Verbindungen gemäß Formel 1. Die erfindungsgemäßen pharmazeutischen Zusammensetzungen können nach einer der bekannten pharmazeutischen Methoden hergestellt werden, die im wesentlichen darin bestehen, dass die Bestandteile mit pharmakologisch verträglichen Träger- und/oder Hilfsstoffen gemischt werden.

Erfindungsgemäße pharmazeutische Zusammensetzungen sind solche, die für orale, rektale, topische, perorale (z.B. sublinguale) und parenterale (z.B. subkutane, intramuskuläre, intradermale oder intravenöse) Verabreichung geeignet sind, wenngleich die geeignetste Verabreichungsweise in jedem Einzelfall von der Art und Schwere des zu behandelnden Zustandes und von der Art der jeweils verwendeten Verbindung gemäß Formel I abhängig ist. Auch dragierte Formulierungen und dragierte Retardformulierungen gehören in den Rahmen der Erfindung. Bevorzugt sind säure- und magensaftresistente Formulierungen. Geeignete magensaftresistente Beschichtungen umfassen Celluloseacetatphthalat, Poylvinylacetatphthalat, Hydroxypropylmethylcellulosephthalat und anionische Polymere von Methacrylsäure und Methacrylsäuremethylester.

Geeignete pharmazeutische Verbindungen für die orale Verabreichung können in separaten Einheiten vorliegen, wie zum Beispiel Kapseln, Oblatenkapseln, Lutschtabletten oder Tabletten, die jeweils eine bestimmte Menge der Verbindung gemäß Formel I enthalten; als Pulver oder Granulate; als Lösung oder Suspension in einer wässrigen oder nicht-wässrigen Flüssigkeit; oder als eine Öl-in-Wasser- oder Wasser-in-Öl-Emulsion. Diese Zusammensetzungen können, wie bereits erwähnt, nach jeder geeigneten pharmazeutischen Methode zubereitet werden, die einen Schritt umfasst, bei dem der Wirkstoff und der Träger (der aus einem oder mehreren zusätzlichen Bestandteilen bestehen kann) in Kontakt gebracht werden. Im allgemeinen werden die Zusammensetzungen durch gleichmäßiges und homogenes Vermischen des Wirkstoffs mit einem flüssigen und/oder feinverteilten festen Träger hergestellt, wonach das Produkt, falls erforderlich, geformt wird. So kann beispielsweise eine Tablette hergestellt werden, indem ein Pulver oder Granulat der Verbindung verpresst oder geformt wird, gegebenenfalls mit einem oder mehreren zusätzlichen Bestandteilen. Gepresste Tabletten können durch tablettieren der Verbindung in frei fließender Form, wie beispielsweise einem Pulver oder Granulat, gegebenenfalls gemischt mit einem Bindemittel, Gleitmittel, inertem Verdünner und/oder einem (mehreren) oberflächenaktiven/dispergierenden Mittel in einer geeigneten Maschine hergestellt werden. Geformte Tabletten können durch Formen der pulverförmigen, mit einem inerten flüssigen Verdünnungsmittel befeuchteten Verbindung in einer geeigneten Maschine hergestellt werden.

Pharmazeutische Zusammensetzungen, die für eine perorale (sublinguale) Verabreichung geeignet sind, umfassen Lutschtabletten, die eine Verbindung gemäß Formel I mit einem Geschmacksstoff enthalten, üblicherweise Saccharose und Gummi arabicum oder Tragant, und Pastillen, die die Verbindung in einer inerten Basis wie Gelatine und Glycerin oder Saccharose und Gummi arabicum umfassen.

Geeignete pharmazeutische Zusammensetzungen für die parenterale Verabreichung umfassen vorzugsweise sterile wässrige Zubereitungen einer Verbindung gemäß Formel 1, die vorzugsweise isotonisch mit dem Blut des vorgesehenen Empfängers sind. Diese Zubereitungen werden vorzugsweise intravenös verabreicht, wenngleich die Verabreichung auch subkutan, intramuskulär oder intradermal als Injektion erfolgen kann. Diese Zubereitungen können vorzugsweise hergestellt werden, indem die Verbindung' mit Wasser gemischt wird und die erhaltene Lösung steril und mit dem Blut isotonisch gemacht wird. Injizierbare erfindungsgemäße Zusammensetzungen enthalten im allgemeinen von 0,1 bis 5 Gew.-% der aktiven Verbindung.

Geeignete pharmazeutische Zusammensetzungen für die rektale Verabreichung liegen vorzugsweise als Einzeldosis-Zäpfchen vor. Diese können hergestellt werden, indem man eine Verbindung gemäß Formel I mit einem oder mehreren herkömmlichen festen Trägern, beispielsweise Kakaobutter, mischt und das entstehende Gemisch in Form bringt.

Geeignete pharmazeutische Zusammensetzungen für die topische Anwendung auf der Haut liegen vorzugsweise als Salbe, Creme, Lotion, Paste, Spray, Aerosol oder Öl vor. Als Träger können Vaseline, Lanolin, Polyethylenglykole, Alkohole und Kombinationen von zwei oder mehreren dieser Substanzen verwendet werden. Der Wirkstoff ist im allgemeinen in einer Konzentration von 0,1 bis 15 Gew.-% der Zusammensetzung vorhanden, beispielsweise von 0,5 bis 2%.

Auch eine transdermale Verabreichung ist möglich. Geeignete pharmazeutische Zusammensetzungen für transdermale. Anwendungen können als einzelne Pflaster vorliegen, die für einen langzeitigen engen Kontakt mit der Epidermis des Patienten geeignet sind. Solche Pflaster enthalten geeigneterweise den Wirkstoff in einer gegebenenfalls gepufferten wässrigen Lösung, gelöst und/oder dispergiert in einem Haftmittel oder dispergiert in einem Polymer. Eine geeignete Wirkstoff-Konzentration beträgt ca. 1 % bis 35%, vorzugsweise ca. 3% bis 15%.. Als eine besondere Möglichkeit kann der Wirkstoff, wie beispielsweise in Pharmaceutical Research, 2(6): 318 (1986) beschrieben, durch Elektrotransport oder lontophorese freigesetzt werden.

Als weitere Wirkstoffe für die Kombinationspräparate sind geeignet:
Alle Antidiabetika, die in der Roten Liste 2001, Kapitel 12 genannt sind. Sie können mit den erfindungsgemäßen Verbindungen der Formel I insbesondere zur synergistischen Wirkungsverbesserung kombiniert werden. Die Verabreichung der Wirkstoffkombination kann entweder durch getrennte Gabe der Wirkstoffe an den Patienten oder in Form von Kombinationspräparaten, worin mehrere Wirkstoffe in einer pharmazeutischen Zubereitung vorliegen, erfolgen. Die meisten der nachfolgend aufgeführten Wirkstoffe sind in USP Dictionary of USAN and International Drug Names, US Pharmacopeia, Rockville 2001, offenbart. Antidiabetika umfassen Insulin und Insulinderivate, wie z.B. Lantus^{®} (siehe www.lantus.com) oder HMR 1964, schnell wirkende Insuline (siehe US 6,221,633), GLP-1-Derivate wie z.B. diejenigen die in WO 98/08871 von Novo Nordisk A/S offenbart wurden, sowie oral wirksame hypoglykämische Wirkstoffe.
Die oral wirksamen hypoglykämischen Wirkstoffe umfassen vorzugsweise Sulphonylfharnstoffe, Biguanidine, Meglitinide, Oxadiazolidindione, Thiazolidindione, Glukosidase-Inhibitoren, Glukagon-Antagonisten, GLP-1-Agonisten, Kaliumkanalöffner, wie z.B. diejenigen, die in WO 97/26265 und WO 99/03861 von Novo Nordisk A/S offenbart wurden, Insulin-Sensitizer, Inhibitoren von Leberenzymen, die an der Stimulation der Glukoneogenese und/oder Glykogenolyse beteiligt sind, Modulatoren der Glukoseaufnahme, den Fettstoffwechsel verändernde Verbindungen wie antihyperlipidämische Wirkstoffe und antilipidämische Wirkstoffe, Verbindungen, die die Nahrungsmitteleinnahme verringern, PPAR- und PXR-Agonisten und Wirkstoffe, die auf den ATP-abhängigen Kaliumkanal der Betazellen wirken.

Bei einer Ausführungsform der Erfindung werden die Verbindungen der Formel I in Kombination mit einem HMGCoA-Reduktase Inhibitor wie Simvastatin, Fluvastatin, Pravastatin, Lovastatin, Atorvastatin, Cerivastatin, Rosuvastatin verabreicht.

Bei einer Ausführungsform der Erfindung werden die Verbindungen der Formel I in Kombination mit einem Cholesterinresorptionsinhibitor, wie z.B. Ezetimibe, Tiqueside, Pamaqueside, verabreicht.

Bei einer Ausführungsform der Erfindung werden die Verbindungen der Formel I in Kombination mit einem PPAR gamma Agonist, wie z.B. Rosiglitazon, Pioglitazon, JTT-501, Gl 262570, verabreicht.

Bei einer Ausführungsform der Erfindung werden die Verbindungen der Formel I in Kombination mit PPAR alpha Agonist, wie z.B. GW 9578, GW 7647, verabreicht.

Bei einer Ausführungsform der Erfindung werden die Verbindungen der Formel I in Kombination mit einem gemischten PPAR alpha/gamma Agonisten, wie z.B. GW 1536, AVE 8042, AVE 8134, AVE 0847, oder wie in PCT/US 11833, PCT/US 11490, DE10142734.4 beschrieben verabreicht.

Bei einer Ausführungsform der Erfindung werden die Verbindungen der Formel I in Kombination mit einem Fibrat, wie z.B. Fenofibrat, Clofibrat, Bezafibrat, verabreicht.

Bei einer Ausführungsform der Erfindung werden die Verbindungen der Formel I in Kombination mit einem MTP-Inhibitor, wie z.B. Implitapide , BMS-201038, R-103757, verabreicht.

Bei einer Ausführungsform der Erfindung werden die Verbindungen der Formel I in Kombination mit Gallensäureresorptionsinhibitor (siehe z.B. US 6,245,744 oder US 6,221,897), wie z.B. HMR 1741, verabreicht.

Bei einer Ausführungsform der Erfindung werden die Verbindungen der Formel 1 in Kombination mit einem CETP-Inhibitor, wie z.B. JTT-705 , verabreicht.

Bei einer Ausführungsform der Erfindung werden die Verbindungen der Formel I in Kombination mit einem polymeren Gallensäureadsorber, wie z.B. Cholestyramin, Colesevelam, verabreicht.

Bei einer Ausführungsform der Erfindung werden die Verbindungen der Formel I in Kombination mit einem LDL-Rezeptorinducer (siehe US 6,342,512), wie z.B. HMR1171, HMR1586, verabreicht.

Bei einer Ausführungsform der Erfindung werden die Verbindungen der Formel I in Kombination mit einem ACAT-Inhibitor, wie z.B. Avasimibe, verabreicht.

Bei einer Ausführungsform der Erfindung werden die Verbindungen der Formel I in Kombination mit einem Antioxidans, wie z.B. OPC-14117, verabreicht.

Bei einer Ausführungsform der Erfindung werden die Verbindungen der Formel I in Kombination mit einem Lipoprotein-Lipase Inhibitor, wie z.B. NO-1886, verabreicht.

Bei einer Ausführungsform der Erfindung werden die Verbindungen der Formel I in Kombination mit einem ATP-Citrat-Lyase Inhibitor, wie z.B. SB-204990, verabreicht.

Bei einer Ausführungsform der Erfindung werden die Verbindungen der Formel I in Kombination mit einem Squalen Synthetase Inhibitor, wie z.B. BMS-188494, verabreicht.

Bei einer Ausführungsform der Erfindung werden die Verbindungen der Formel I in Kombination mit einem Lipoprotein(a) antagonist, wie z.B. CI-1027 oder Nicotinsäure, verabreicht.

Bei einer Ausführungsform der Erfindung werden die Verbindungen der Formel 1 in Kombination mit einem Lipase Inhibitor, wie z.B. Orlistat, verabreicht.

Bei einer Ausführungsform der Erfindung werden die Verbindungen der Formel I in Kombination mit Insulin verabreicht.
Bei einer Ausführungsform werden die Verbindungen der Formel in Kombination mit einem Sulphonylharnstoff, wie z.B. Tolbutamid, Glibenclamid, Glipizid oder Glimepirid verabreicht.
Bei einer Ausführungsform werden die Verbindungen der Formel I in Kombination mit einem Biguanid, wie z.B. Metformin, verabreicht.

Bei wieder einer Ausführungsform werden die Verbindungen der Formel I in Kombination mit einem Meglitinid, wie z.B. Repaglinid, verabreicht.
Bei einer Ausführungsform werden die Verbindungen der Formel I in Kombination mit einem Thiazolidindion, wie z.B. Troglitazon, Ciglitazon, Pioglitazon, Rosiglitazon oder den in WO 97/41097 von Dr. Reddy's Research Foundation offenbarten Verbindungen, insbesondere 5-[[4-[(3,4-Dihydro-3-methyl-4-oxo-2-chinazolinylmethoxy]-phenyl]methyl]-2,4-thiazolidindion, verabreicht.
Bei einer Ausführungsform werden die Verbindungen der Formel I in Kombination mit einem a-Glukosidase-Inhibitor, wie z.B. Miglitol oder Acarbose, verabreicht.
Bei einer Ausführungsform werden die Verbindungen der Formel I in Kombination mit einem Wirkstoff verabreicht, der auf den ATP-abhängigen Kaliumkanal der Betazellen wirkt, wie z.B. Tolbutamid, Glibenclamid, Glipizid, Glimepirid oder Repaglinid.
Bei einer Ausführungsform werden die Verbindungen der Formel 1 in Kombination mit mehr als einer der vorstehend genannten Verbindungen, z.B. in Kombination mit einem Sulphonylharnstoff und Metformin, einem Sulphonylharnstoff und Acarbose, Repaglinid und Metformin, Insulin und einem Sulphonylharnstoff, Insulin und Metformin, Insulin und Troglitazon, Insulin und Lovastatin, etc. verabreicht.

Bei einer weiteren Ausführungsform werden die Verbindungen der Formel I in Kombination mit CART-Modulatoren (siehe "Cocaine-amphetamine-regulated transcript influences energy metabolism, anxiety and gastric emptying in mice" Asakawa, A, et al., M.: Hormone and Metabolic Research (2001), 33(9), 554-558), NPY-Antagonisten z.B. Naphthalin-1-sulfonsäure-{4-[(4-amino-quinazolin-2-ylamino)-methyl]-cyclohexylmethyl}-amid Hydrochlorid (CGP 71683A)), MC4-Agonisten (z.B. 1-Amino-1,2,3,4-tetrahydro-naphthalin-2-carbonsäure [2-(3a-benzyl-2-methyl-3-oxo-2,3,3a,4,6,7-hexahydro-pyrazolo[4,3-c]pyridin-5-yl)-1-(4-chloro-phenyl)-2-oxo-ethyl]-amid; (WO 01/91752)), Orexin-Antagonisten (z.B. 1-(2-Methyl-benzoxazol-6-yl)-3-[1,5]naphthyridin-4-yl-harnsfoff Hydrochlorid (SB-334867-A)), H3-Agonisten (3-Cyclohexyl-1-(4,4-dimethyl-1,4,6,7-tetrahydro-imidazo[4,5-c]pyridin-5-yl)-propan-1-on Oxalsäuresalz (WO 00/63208)); TNF-Agonisten, CRF-Antagonisten (z.B. [2-Methyl-9-(2,4,6-trimethyl-phenyl)-9H-1,3,9-triaza-fluoren-4-yl]-dipropyl-amin (WO 00/66585)), CRF BP-Antagonisten (z.B. Urocortin), Urocortin-Agonisten, β3-Agonisten (z.B. 1-(4-Chloro-3-methanesulfonylmethyl-phenyl)-2-[2-(2,3-dimethyl-1 H-indol-6-yloxy)-ethylamino]-ethanol Hydrochlorid (WO 01/83451)), MSH (Melanocyt-stimulierendes Hormon)-Agonisten, CCK-A Agonisten (z.B. {2-[4-(4-Chloro-2,5-dimethoxy-phenyl)-5-(2-cyclohexyl-ethyl)-thiazol-2-ylcarbamoyl]-5,7-dimethyl-indol-1-yl}-essigsäure Trifluoressigsäuresalz (WO, 99/15525)), Serotonin-Wiederaufnahme-Inhibitoren (z.B. Dexfenfluramine), gemischte Sertonin- und noradrenerge Verbindungen (z.B. WO 00/71.549), 5HT-Agonisten z.B. -1-(3-Ethyl-benzofuran-7-yl)-piperazin Oxalsäuresalz (WO 01/09111), Bombesin-Agonisten, Galanin-Antagonisten, Wachstumshormon (z.B. humanes Wachstumshormon), Wachstumshormon freisetzende Verbindungen (6-Benzyloxy-1 -(2-diisopropylamino-ethylcarbamoyl)-3,4-dihydro-1 H-isochinolin-2-carbonsäuretertiärbutylester (WO 01/85695)), TRH-Agonisten (siehe z.B. EP 0 462 884) entkoppelnde Protein 2- oder 3-Modulatoren, Leptinagonisten (siehe z.B. Lee, Daniel W.; Leinung, Matthew C.; Rozhavskaya-Arena, Marina; Grasso, Patricia. Leptin agonists as a potential approach to the treatment of obesity. Drugs of the Future (2001), 26(9), 873-881), DA-Agonisten (Bromocriptin, Doprexin), Lipase/Amylase-Inhibitoren (z.B. WO 00/40569), PPAR-Modulatoren (z.B. WO 00/78312), RXR-Modulatoren oder TR-β-Agonisten verabreicht.

Bei einer Ausführungsform der Erfindung ist der weitere Wirkstoff Leptin; siehe z.B. "Perspectives in the therapeutic use of leptin", Salvador, Javier, Gomez-Ambrosi, Javier; Fruhbeck, Gema, Expert Opinion on Pharmacotherapy (2001), 2(10), 1615-1622.

Bei einer Ausführungsform ist der weitere Wirkstoff Dexamphatamin oder Amphetamin.
Bei einer Ausführungsform ist der weitere Wirkstoff Fenfluramin oder Dexfenfluramin. Bei noch einer Ausführungsform ist der weitere Wirkstoff Sibutramin.
Bei einer Ausführungsform ist der weitere Wirkstoff Orlistat.
Bei-einer Ausführungsform ist der weitere Wirkstoff Mazindol oder Phentermin.

Bei einer Ausführungsform werden die Verbindungen der Formel I in Kombination mit Ballaststoffen, vorzugsweise unlöslichen Ballaststoffen (siehe z.B. Carob/ Caromax^{®} (Zunft H J; et al., Carob pulp preparation for treatment of hypercholesterolemia, ADVANCES IN THERAPY (2001 Sep-Oct), 18(5), 230-6.) Caromax ist ein Carob enthaltendes Produkt der Fa. Nutrinova, Nutrition Specialties & Food Ingredients GmbH, Industriepark Höchst, 65926 Frankfurt / Main)) verabreicht. Die Kombination mit Caromax^{®} kann in einer Zubereitung erfolgen, oder durch getrennte Gabe von Verbindungen der Formel I und Caromax^{®}. Caromax^{®} kann dabei auch in Form von Lebensmitteln, wie z.B. in Backwaren oder Müsliriegeln, verabreicht werden.

Es versteht sich, dass jede geeignete Kombination der erfindungsgemäßen Verbindungen mit einer oder mehreren der vorstehend genannten Verbindungen und wahlweise einer oder mehreren weiteren pharmakologisch wirksamen Substanzen als unter den Schutzbereich der vorliegenden Erfindung fallend angesehen wird.

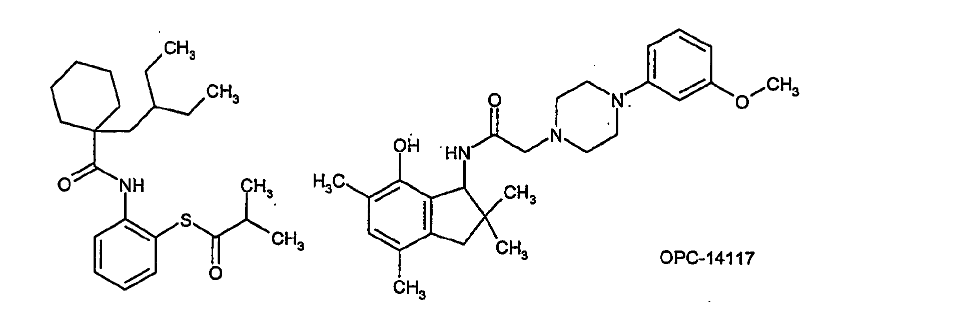

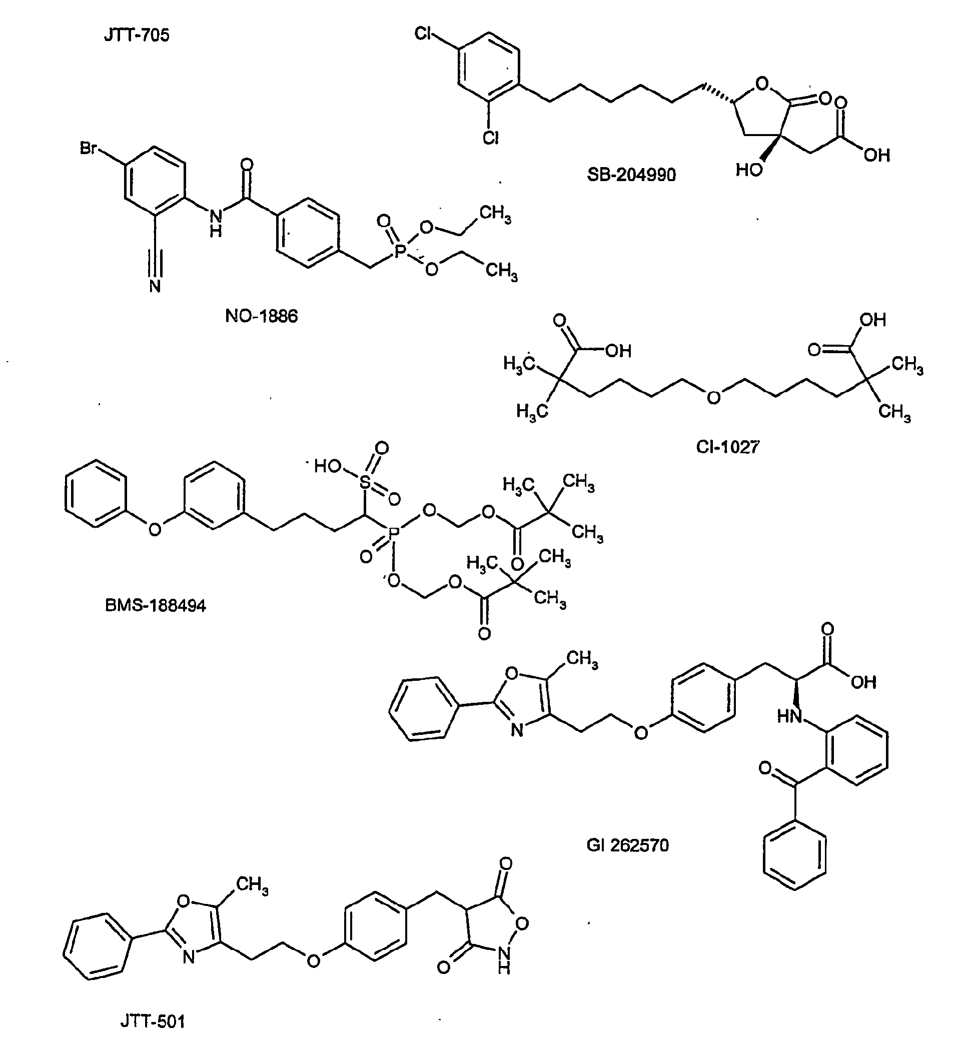

Die nachfolgend aufgeführten Beispiele dienen zur Erläuterung der Erfindung, ohne diese jedoch einzuschränken.

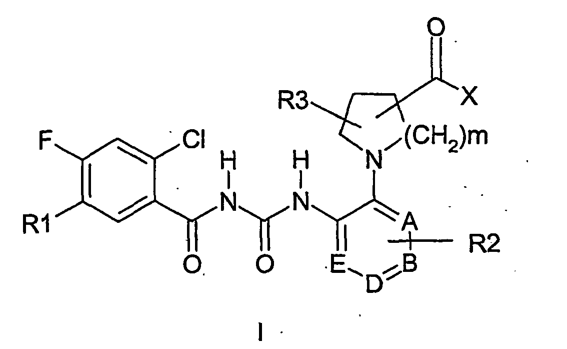

| **Bsp.** | **R1** | **R2** | **R3** | **A** | **B** | **D** | **E** | **-(C=O)-X** | **m** | **Fp.** |
|---|---|---|---|---|---|---|---|---|---|---|
| 1 | H | H | H | N | CH | CH | CH | 4-COOH | 2 | 220,2 |
| 2 | F | H | H | N | CH | CH | CH | 4-COOH | 2 | 229,5 |
| 3 | F | H | H | N | CH | CH | CH | 4-COOMe | 2 | 195,9 |
| 4 | F | H | H | N | CH | CH | CH | 4-CONH2 | 2 | 215,9 |
| 5 | F | H | H | N | CH | CH | CH | 4-CONHCH₂CH₂OH | 2 | 202,8 |
| 6 | F | H | 4-Ph | N | CH | CH | CH | 4-COOH | 2 | 250,9 |

Die Wirksamkeit der Verbindungen wurde wie folgt getestet:

### Glykogenphosphorylase a Aktivitätstest

Der Effekt von Verbindungen auf die Aktivität der aktiven Form der Glykogenphosphorylase (GPa) wurde in der umgekehrten Richtung, durch Verfolgen der Glykogensynthese aus Glukose-1-Phosphat an Hand der Bestimmung der Freisetzung von anorganischem Phosphat, gemessen. Alle Reaktionen wurden als Doppelbestimmungen in Mikrotiterplatten mit 96-Vertiefungen (Half Area Plates, Costar Nr. 3696) durchgeführt, wobei die Änderung der Absorption auf Grund der Bildung des Reaktionsprodukts bei der weiter unten spezifizierten Wellenlänge in einem Multiskan Ascent-Elisa Reader (Lab Systems, Finnland) gemessen wurde.
Um die GPa Enzymaktivität in der umgekehrten Richtung zu messen, wurde die Umwandlung von Glukose-1-Phosphat in Glykogen und anorganisches Phosphat nach der allgemeinen Methode von Engers et al. (Engers HD, Shechosky S, Madsen NB, Can J Biochem 1970 Jul;48(7):746-754) mit folgenden Modifikationen gemessen: Humane Glykogenphosphorylase a (zum Beispiel mit 0,76 mg Protein / ml (Aventis Pharma Deutschland GmbH), gelöst in Pufferlösung E (25 mM β-Glyzerophosphat, pH 7,0, 1 mM EDTA und 1 mM Dithiotreitol) wurde mit Puffer T (50 mM Hepes, pH 7,0, 100 mM KCI, 2,5 mM EDTA, 2,5 mM MgCl₂6H₂O) und Zusatz von 5 mg/ml Glykogen auf eine Konzentration von 10 µg Protein/ml verdünnt. Prüfsubstanzen wurden als 10 mM Lösung in DMSO zubereitet und auf 50 µM mit Pufferlösung T verdünnt. Zu 10 µl dieser Lösung wurden 10 µl 37,5 mM Glukose, gelöst in Pufferlösung T und 5 mg/mL Glykogen, sowie 10 µl einer Lösung von humaner Glykogenphosphorylase a (10 µg Protein/ml) und 20 µl Glukose-1-Phosphat, 2,5 mM zugegeben. Der basale Wert der Glykogenphosphorylase a Aktivität in Abwesenheit von Prüfsubstanz wurde durch Zugabe von 10 µl Pufferlösung T (0,1 % DMSO) bestimmt. Die Mischung wurde 40 Minuten bei Raumtemperatur inkubiert und das freigesetzte anorganische Phosphat mittels der allgemeinen Methode von Drueckes et al. (Drueckes P, Schinzel R, Palm D, Anal Biochem 1995 Sep 1;230(1):173-177) mit folgenden Modifikationen gemessen: 50 µl einer Stop-Lösung von 7,3 mM Ammoniummolybdat, 10,9 mM Zinkacetat, 3,6 % Askorbinsäure, 0,9 % SDS werden zu 50 µl der Enzymmischung gegeben. Nach 60 Minuten Inkubation bei 45 °C wurde die Absorption bei 820 nm gemessen. Zur Bestimmung der Hintergrundsabsorption wurde in einem separaten Ansatz die Stop-Lösung unmittelbar nach Zugabe der Glukose-1-Phosphatlösung zugegeben.
Dieser Test wurde mit einer Konzentrationen von 10 µM der Prüfsubstanz durchgeführt, um die jeweilige Hemmung der Glykogenphosphorylase a in vitro durch die Prüfsubstanz zu bestimmen.

**Tabelle 2: Biologische Aktivität**

| **Bsp.** | **IC-50 (µM)** |
|---|---|
| 1 | 0,04 |
| 2 | 0,01 |
| 4 | 0,16 |
| 5 | 3,65 |

Aus der Tabelle ist abzulesen, dass die Verbindungen der Formel I die Aktivität der Glykogenphosphorylase a hemmen und dadurch zur Senkung des Blutzuckerspiegels gut geeignet sind.

Nachfolgend wird die Herstellung einiger Beispiele detailliert beschrieben, die übrigen Verbindungen der Formel 1 wurden analog erhalten:

### Experimenteller Teil:

### Beispiel 1:

### a) 3'- Nitro-3,4,5,6-tetrahydro-2H-[1,2']bipyridinyl-4-carbonsäure

Die Mischung bestehend aus 634 mg 2-Chlor-3-nitro-pyridin, 620 mg Piperidincarbonsäure, 820 mg Pottasche und 3 ml NMP wurde 2 Stunden bei 80°C gerührt. Nach dem Erkaltenlassen wurden 30 ml Wasser zugefügt und das Rohprodukt mit 30 ml Essigester extrahiert. Nach dem Trocknen der organischen Phase über Natriumsulfat wurde im Vakuum eingeengt.
Ausbeute: 850 mg Fp.: Öl (roh)
Analog wurden hergestellt:
3'-Nitro-4-phenyl-3,4,5,6-tetrahydro-2H-[1,2']bipyridinyl-4-carbonsäure (Fp.: Öl)
3'-Nitro-3,4,5,6-tetrahydro-2H-[1,2']bipyridinyl-4-carbonsäuremethylester (FP.: Öl)

### b) 3'-Amino-3,4,5,6-tetrahydro-2H-[1,2']bipyridinyl-4-carbonsäure

Die Lösung von 850 mg 3'-Nitro-3,4,5,6-tetrahydro-2H-[1,2']bipyridinyl-4-carbosäure in 40 ml Essigester wurde mit 2,3 g Zinn-2-chlorid versetzt und 3 Stunden bei Raumtemperatur gerührt. Dann wurde mit 50 ml Wasser ausgeschüttelt, die wässrige Phase mit Natriumdihydrogenphosphat auf pH = 6 gestellt und das Produkt mit Essigester ausgeschüttelt. Nach dem Trocknen der organische Phase über Natriumsulfat wurde im Vakuum eingeengt.
Ausbeute: 480 mg Fp. Harz
Analog wurden hergestellt:
3'-Amino-3,4,5,6-tetrahydro-2H-[1,2']bipyridinyl-4-carbonsäuremethylester (Fp.: Harz)
3'-Amino-4-phenyl-3,4,5,6-tetrahydro-2H-[1,2']bipyridinyl-4-carbonsäure (Fp.: 201,4°C)

### c) 3'-[3-(2-Chlor-4-fluor-benzoyl)-ureido]-3,4,5,6-tetrahydro-2H-[1,2]bipyridinyl-4- carbonsäure

In die Lösung von 100 mg 3'-Amino-3,4,5,6-tetrahydro-2H-(1,2")bipyridinyl-4-carbonsäure in 5 ml Acetonitril wurde unter Umrühren die äquimolare Lösung von 2-Chlor-4-fluor-benzoylisocyanat in Acetonitril getropft. Die Mischung wurde 6 bei RT gerührt und der Niederschlag abgesaugt und im Vakuum bei RT getrocknet.
Ausbeute: 105 mg Fp.: 220,2°C

### Beispiel 2:

### 3'-[3-(2-Chlor-4,5-difluor-benzoyl)-ureido]-3,4,5,6-tetrahydro-2H-[1,2']bipyridinyl-4-carbonsäure

### Beispiel 3:

### 3'-[3-(2-Chlor-4,5-difluor-benzoyl)-ureido]-3,4,5,6-tetrahydro-2H-[1,2']bipyridinyl-4-carbonsäure-methylester

### Beispiel 4:

### 3'-[3-(2-Chlor-4,5-difluor-benzoyl)-ureido]-3,4,5,6-tetrahydro-2H-[1,2']bipyridinyl-4-carbonamid

Die Mischung bestehend aus 50 mg 3'-[3-(2-Chlor-4,5-difluor-benzoyl)-ureido]-3,4,5,6-tetrahydro-2H-[1,2']bipyridinyl-4-carbonsäure, 0,5 ml DMF, 6,1 mg Ammoniumchlorid, 40 µl Hünigbase und 37,3 mg Totu wurde 2 Stunden bei Raumtemperatur gerührt. Danach wurde mit 5 ml Wasser verdünnt und die Mischung kurz verrührt. Der Niederschlag wurde abgesaugt und im Vakuum getrocknet.
Ausbeute: 43 mg Fp.: 215,9°C

### Beispiel 5:

### 3'-[3-(2-Chlor-4,5-difluor-benzoyl)-ureido]-3,4,5,6-tetrahydro-2H-[1,2']bipyridinyl-4-carbonsäure-(2-hydroxi-ethyl)-amid (A003105307)

wurde analog Beispiel 4 durch Ersatz von Ammoniumchlorid durch 2-Aminoethanol erhalten. Fp.: 202,8°C

Beispiel 6:

**3'-[3-(2-Chlor-4,5-difluor-benzoyl)-ureido]-4-phenyl-3,4,5,6-tetrahydro-2H-[1,2']bipyridinyl-4-carborisäure**

## Patentansprüche

1. Verbindungen der Formel I , 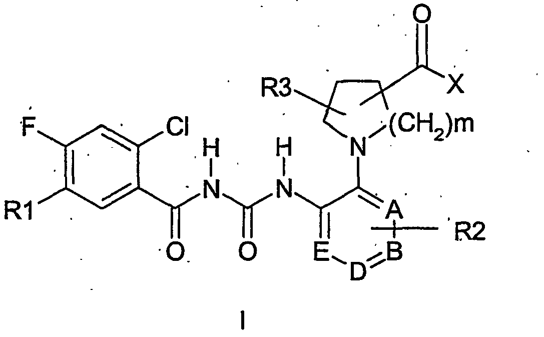 worin bedeuten
R1, R2 unabhängig voneinander H, F, Cl, Br, (C₁-C₆)-Alkyl, CF₃, OCF₃, NO₂, CN, O-(C₁-C₆)-Alkyl, COO(C₁-C₆)-Alkyl, COOH, CO-(C₁-C₆)-Alkyl, (C₀-C₆)-Alkyl-COOH, (C₀-C₆)-Alkyl -COO(C₁-C₆)-Alkgl, SO₂-(C₁-C₆)-Alkyl;
R3 H, (C₁-C₆)-Alkyl, (C₀-C₆)-Alkyl-Aryl, O-(C₁-C₆)-Alkyl, O-(C₂-C₆)-Alkenyl, O-(C₂-C₆)-Alkinyl, wobei die Alkyl-, Aryl-, Alkenyl- und Alkinylreste ein oder mehrfach durch F, CI oder Br substituiert sein können;
X OH, O-(C₁-C₆)-Alkyl, NH₂, NH(C₁-C₆)-Alkyl, N((C₁-C₆)-Alkyl)₂;
A, B, D, E unabhängig voneinander CH oder N, wobei mindestens eine der Gruppen A, B, D und E die Bedeutung N hat;
m 0, 1, 2;
sowie deren physiologisch verträgliche Salze.

2. Verbindungen der Formel I, gemäß Anspruch 1, **dadurch gekennzeichnet, daß** darin bedeuten
R1, R2 unabhängig voneinander H, F, CI, Br, (C₁-C₆)-Alkyl, CF₃, OCF₃, NO₂, CN, O-(C₁-C₆)-Alkyl, COO(C₁-C₆)-Alkyl, COOH, CO-(C₁-C₆)-Alkyl, (C₀-C₆)-Alkyl-COOH, (C₀-C₆)-Alkyl-COO(C₁-C₆)-Alkyl; SO₂-(C₁-C₆)-Alkyl;
R3 H, (C₁-C₆)-Alkyl, (C₀-C₆)-Alkyl-Aryl, O-(C₁-C₆)-Alkyl, O-(C₂-C₆)-Alkenyl, O-(C₂-C₆)-Alkinyl, wobei die Alkyl-, Aryl-, Alkenyl- und Alkinylreste ein oder mehrfach durch F, CI oder Br substituiert sein können;
X OH, O-(C₁-C₆)Alkyl, NH₂, NH(C₁-C₆)-Alkyl, N((C₁-C₆)-Alkyl);
A, B, D, E unabhängig voneinander CH oder N, wobei mindestens eine der Gruppen A, B, D und E die Bedeutung N hat;
m 1, 2;
sowie deren physiologisch verträgliche Salze.

3. Verbindungen der Formel 1, gemäß Anspruch 1 oder 2, **dadurch gekennzeichnet, daß** darin bedeuten
R1 H, F;
R2 unabhängig voneinander H, F, Cl, Br, (C₁-C₆)-Alkyl, CF₃, OCF₃, O-(C₁-C₆)-Alkyl, COO(C₁-C₆)-Alkyl, COOH, CO-(C₁-C₆)-Alkyl, (C₀-C₆)-Alkyl-COOH, (C₀-C₆)-Alkyl-COO(C₁-C₆)-Alkyl, SO₂-(C₁-C₆)-Alkyl;
R3 H, (C₁-C₆)-Alkyl, (C₀-C₆)-Alkyl-Aryl, O-(C₁-C₆)-Alkyl, O-(C₂-C₆)-Alkenyl, O-(C₂-C₆)-Alkinyl, wobei die Alkyl-, Aryl-, Alkenyl- und Alkinylreste ein oder mehrfach durch F, CI oder Br substituiert sein können;
X OH, O-(C₁-C₆)-Alkyl, NH₂, NH(C₁-C₆)-Alkyl, N((C₁-C₆)-Alkyl)2;
A N
B, D, E CH
m 1, 2;
sowie deren physiologisch verträgliche Salze.

4. Verbindungen der Formel I, gemäß einem oder mehreren der Ansprüche 1 bis 3, **dadurch gekennzeichnet, daß** darin bedeuten
R1 H, F;
R2 H, Cl, (C₁-C₆)-All<yl, CF₃, COO(C₁-C₆)-Alkyl, COOH,
R3 H, Phenyl;
X OH, O-(C₁-C₆)-Alkyl, NH₂, NH(C₁-C₆)-Alkyl, N((C₁-C₆)-Alkyl)₂;
A N;
B, D, E CH;
m 2;
sowie deren physiologisch verträgliche Salze.

5. Arzneimittel enthaltend eine oder mehrere der Verbindungen gemäß einem oder mehreren der Ansprüche 1 bis 4.

6. Arzneimittel enthaltend eine oder mehrere der Verbindungen gemäß einem oder mehreren der Ansprüche 1 bis 4 und mindestens einen weiteren Wirkstoff.

7. Arzneimittel, gemäß Anspruch 6, **dadurch gekennzeichnet, daß** es als weiteren Wirkstoff eine oder mehrere
Antidiabetika, hypoglykämischen Wirkstoffe, HMGCoA-Reduktase Inhibitoren, Cholesterinresorptionsinhibitoren, PPAR gamma Agonisten, PPAR alpha Agonisten, PPAR alpha/gamma Agonisten, Fibrate, MTP-Inhibitoren, Gallensäureresorptionsinhibitoren, CETP-Inhibitoren, polymere Gallensäureadsorber, LDL-Rezeptorinducer, ACAT-Inhibitoren, Antioxidantien, Lipoprotein-Lipase Inhibitoren, ATP-Citrat-Lyase Inhibitoren, Squalen synthetase inhibitoren, Lipoprotein(a) antagonisten, Lipase Inhibitoren, Insuline, Sulphonylharnstoffe, Biguanide, Meglitinide, Thiazolidindione, α-Glukosidase-Inhibitoren, auf den ATP-abhängigen Kaliumkanal der Betazellen wirkende Wirkstoffe, CART-Agonisten, NPY-Agonisten, MC4-Agonisten, Orexin-Agonisten, H3-Agonisten, TNF-Agonisten, CRF-Agonisten, CRF BP-Antagonisten, Urocortin-Agonisten, β3-Agonisten, MSH (Melanocyt-stimulierendes Hormon)-Agonisten, CCK-Agonisten, Serotonin-Wiederaufnahme-Inhibitoren, gemischte Sertonin- und noradrenerge Verbindungen, 5HT-Agonisten, Bombesin-Agonisten, Galanin-Antagonisten, Wachstumshormone, Wachstumshormon freisetzende Verbindungen, TRH-Agonisten, entkoppelnde Protein 2- oder 3-Modulatoren, Leptinagonisten, DA-Agonisten (Bromocriptin, Doprexin), Lipase/Amylase-Inhibitoren, PPAR-Modulatoren, RXR-Modulatoren oder TR-β-Agonisten oder Amphetamine enthält.

8. Verwendung der Verbindungen gemäß einem oder mehreren der Ansprüche 1 bis 4 zur Herstellung eines Medikamentes zur Blutzuckersenkung.

9. Verwendung der Verbindungen gemäß einem oder mehreren der Ansprüche 1 bis 4 zur Herstellung eines Medikamentes zur Behandlung des Typ II Diabetes.

10. Verwendung der Verbindungen gemäß einem oder mehreren der Ansprüche 1 bis 4 zur Herstellung eines Medikamentes zur Behandlung von Lipid- und Kohlenhydratstoffwechselstörungen.

11. Verwendung der Verbindungen gemäß einem oder mehreren der Ansprüche 1 bis 4 zur Herstellung eines Medikaments zur Behandlung arteriosklerotischer Erscheinungen.

12. Verwendung der Verbindungen gemäß einem oder mehreren der Ansprüche 1 bis 4 zur Herstellung eines Medikaments zur Behandlung von Insulin Resistenz.

13. Herstellung der Verbindungen der Formel I gemäß einem oder mehreren der Ansprüche 1 bis 4, **dadurch gekennzeichnet, dass** Harnstoffe der Formel 2 oder Anilinderivate der Formel 3 mit Aroyl-isocyanaten oder mit reaktiven Säurederivaten, wie Säurechloriden oder Anhydriden, der Formel 4 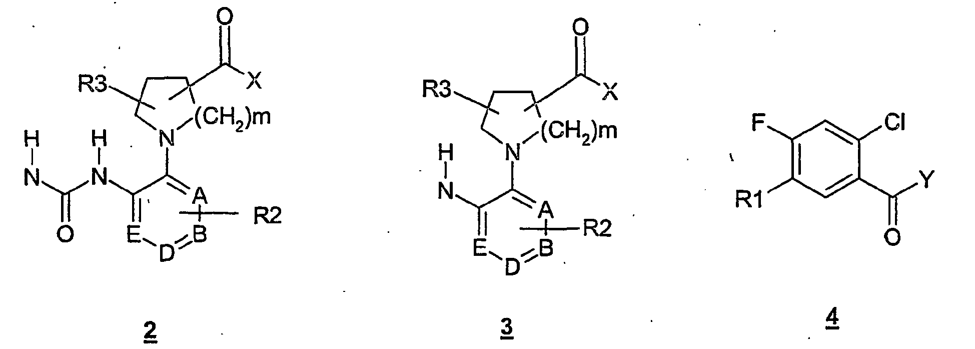 worin R1 bis R3 und A, B, D und E die in Anspruch 1 angegebenen Bedeutungen haben, umgesetzt werden.

14. Verfahren zur Herstellung eines Arzneimittels enthaltend eine oder mehrere der Verbindungen gemäß einem oder mehreren der Ansprüche 1 bis 4, **dadurch gekennzeichnet, daß** der Wirkstoff mit einem pharmazeutisch geeigneten Träger vermischt wird und diese Mischung in eine für die Verabreichung geeignete Form gebracht wird.

## Claims

1. Compounds of the formula I 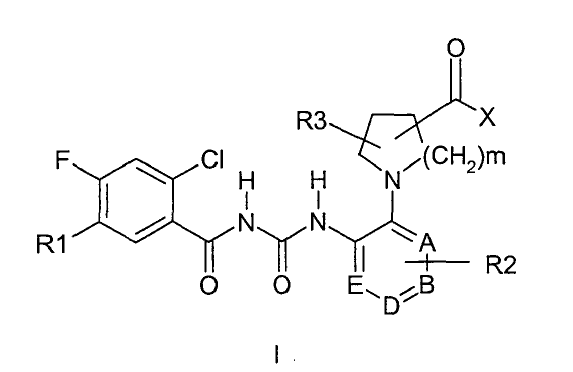 where
R1, R2 are each independently H, F, CI, Br, (C₁-C₆)-alkyl, CF₃, OCF₃, NO₂, CN, O-(C₁-C₆)-alkyl, COO(C₁-C₆)-alkyl, COOH, CO-(C₁-C₆)-alkyl, (C₀-C₆)-alkyl-COOH, (C₀-C₆)-alkyl -COO(C₁-C₆)-alkyl, SO₂-(C₁-C₆)-alkyl;
R3 is H, (C₁-C₆)-alkyl, (C₀-C₆)-alkyl-aryl, O-(C₁-C₆)-alkyl, O-(C₂-C₆)-alkenyl, O-(C₂-C₆)-alkynyl, where the alkyl, aryl, alkenyl and alkynyl radicals may be mono- or polysubstituted by F, CI or Br;
X is OH, O-(C₁-C₆)-alkyl, NH₂, NH(C₁-C₆)-alkyl, N((C₁-C₆)-alkyl)₂;
A, B, D and E are each independently CH or N, and at least one of the groups A, B, D and E has the meaning N;
m is 0, 1, 2;
and their physiologically tolerated salts.

2. Compounds of the formula I as claimed in claim 1, wherein
R1, R2 are each independently H, F, Cl, Br, (C₁-C₆)-alkyl, CF₃, OCF₃, NO₂, CN, O-(C₁-C₆)-alkyl, COO(C₁-C₆)-alkyl, COOH, CO-(C₁-C₆)-alkyl, (C₀-C₆)-alkyl-COOH, (C₀-C₆)-alkyl-COO(C₁-C₆)-alkyl, SO₂-(C₁-C₆)-alkyl;
R3 is H, (C₁-C₆)-alkyl, (C₀-C₆)-alkyl-aryl, O-(C₁-C₆)-alkyl, O-(C₂-C₆)-alkenyl, O-(C₂-C₆)-alkynyl, where the alkyl, aryl, alkenyl and alkynyl radicals may be mono- or polysubstituted by F, CI or Br;
X is OH, O-(C₁-C₆)-alkyl, NH₂, NH(C₁-C₆)-alkyl, N((C₁-C₆)-alkyl)₂;
A, B, D and E are each independently CH or N, and at least one of the groups A, B, D and E has the meaning N;
m is 1, 2;
and their physiologically tolerated salts.

3. Compounds of the formula I as claimed in claim 1 or 2, wherein
R1 is H, F;
R2 is each independently H, F, Cl, Br, (C₁-C₆)-alkyl, CF₃, OCF₃, O-(C₁-C₆)-alkyl, COO(C₁-C₆)-alkyl, COOH, CO-(C₁-C₆)-alkyl, (C₀-C₆)-alkyl-COOH, (C₀-C₆)-alkyl-COO(C₁-C₆)-alkyl, SO₂-(C₁-C₆)-alkyl;
R3 is H, (C₁-C₆)-alkyl, (C₀-C₆)-alkyl-aryl, O-(C₁-C₆)-alkyl, O-(C₂-C₆)-alkenyl, O-(C₂-C₆)-alkynyl, where the alkyl, aryl, alkenyl and alkynyl radicals may be mono- or polysubstituted by F, Cl or Br;
X is OH, O-(C₁-C₆)-alkyl, NH₂, NH(C₁-C₆)-alkyl, N((C₁-C₆)-alkyl)₂;
A is N
B, D, E are CH
m is 1, 2;
and their physiologically tolerated salts.

4. Compounds of the formula I as claimed in one or more of claims 1 to 3, wherein
R1 is H, F;
R2 is H, CI, (C₁-C₆)-alkyl, CF₃, COO(C₁-C₆)-alkyl, COOH,
R3 is H, phenyl;
X is OH, O-(C₁-C₆)-alkyl, NH₂, NH(C₁-C₆)-alkyl, N((C₁-C₆)-alkyl)₂;
A is N;
B, D, E are CH;
m is 2;
and their physiologically tolerated salts.

5. A pharmaceutical comprising one or more of the compounds as claimed in one or more of claims 1 to 4.

6. A pharmaceutical comprising one or more of the compounds as claimed in one or more of claims 1 to 4 and at least one further active ingredient.

7. A pharmaceutical as claimed in claim 6, which comprises, as a further active ingredient, one or more
antidiabetics, hypoglycemic active ingredients, HMG-CoA reductase inhibitors, cholesterol absorption inhibitors, PPAR gamma agonists, PPAR alpha agonists, PPAR alpha/gamma agonists, fibrates, MTP inhibitors, bile acid absorption inhibitors, CETP inhibitors, polymeric bile acid adsorbents, LDL receptor inducers, ACAT inhibitors, antioxidants, lipoprotein lipase inhibitors, ATP-citrate lyase inhibitors, squalene synthetase inhibitors, lipoprotein(a) antagonists, lipase inhibitors, insulins, sulfonylureas, biguanides, meglitinides, thiazolidinediones, α-glucosidase inhibitors, active ingredients acting on the ATP-dependent potassium channel of the beta cells, CART agonists, NPY agonists, MC4 agonists, orexin agonists, H3 agonists, TNF agonists, CRF agonists, CRF BP antagonists, urocortin agonists, β3 agonists, MSH (melanocyte-stimulating hormone) agonists, CCK agonists, serotonin reuptake inhibitors, mixed serotoninergic and noradrenergic compounds, 5HT agonists, bombesin agonists, galanin antagonists, growth hormones, growth hormone-releasing compounds, TRH agonists, uncoupling protein 2 or 3 modulators, leptin agonists, DA agonists (bromocriptine, Doprexin), lipase/amylase inhibitors, PPAR modulators, RXR modulators or TR-β agonists or amphetamines.

8. The use of the compounds as claimed in one or more of claims 1 to 4 for producing a medicament for blood sugar reduction.

9. The use of the compounds as claimed in one or more of claims 1 to 4 for producing a medicament for treating type II diabetes.

10. The use of the compounds as claimed in one or more of claims 1 to 4 for producing a medicament for treating lipid and carbohydrate metabolism disorders.

11. The use of the compounds as claimed in one or more of claims 1 to 4 for producing a medicament for treating arteriosclerotic symptoms.

12. The use of the compounds as claimed in one or more of claims 1 to 4 for producing a medicament for treating insulin resistance.

13. The preparation of the compounds of the formula I as claimed in one or more of claims 1 to 4, which comprises reacting ureas of the formula 2 or aniline derivatives of the formula 3 with aroyl isocyanates or with reactive acid derivatives, such as acid chlorides or anhydrides, of the formula 4 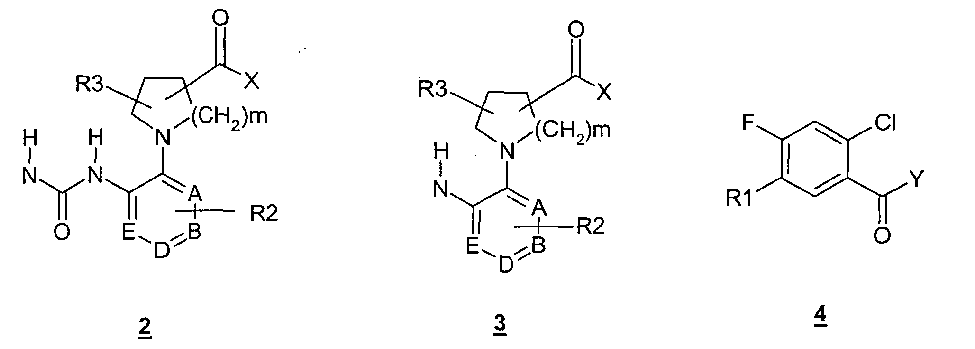 where R1 to R3 and A, B, D and E are each as defined in claim 1.

14. A process for producing a pharmaceutical comprising one or more of the compounds as claimed in one or more of claims 1 to 4, which comprises mixing the active ingredient with a pharmaceutically suitable carrier and converting this mixture into a form suitable for administration.

## Revendications

1. Composés de formule 1 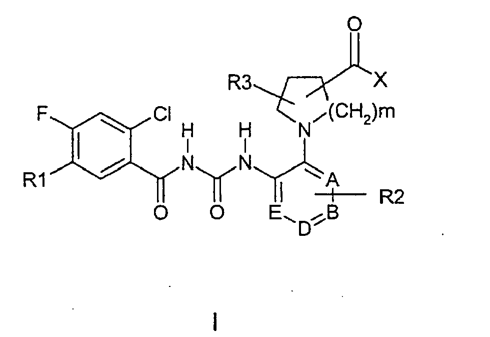 dans laquelle
R1, R2 sont chacun indépendamment H, F, CI, Br, alkyl(C₁-C₆), CF₃, OCF₃, NO₂, CN, O-alkyl(C₁-C₆), COO-alkyl(C₁-C₆), COOH, CO-alkyl(C₁-C₆), alkyl(C₀-C₆)-COOH, alkyl(C₀-C₆)-COO-alkyl(C₁-C₆), SO₂-alkyl(C₁ -C₆) ;
R3 est H, alkyl(C₁-C₆), alkyl(C₀-C₆)-aryle, O-alkyl(C₁-C₆), O-alcényl(C₂-C₆), O-alcynyi(C₂-C₆), où les radicaux alkyle, aryle, alcényle et alcynyle peuvent être mono- ou polysubstitués par F, CI ou Br ;
X est OH, O-alkyl(C₁-C₆), NH₂, NH-alkyl(C₁-C₆), N(alkyl(C₁-C₆))2 ;
A, B, D et E sont chacun indépendamment CH ou N, et au moins un parmi les groupements A, B, D et E a pour signification N ;
m vaut 0, 1,2;
et leurs sels physiologiquement tolérés.

2. Composés de formule 1 selon la revendication 1, **caractérisés en ce que**
R1, R2 sont chacun indépendamment H, F, CI, Br, alkyl(C₁-C₆), CF₃, OCF₃, NO₂, CN, O-alkyl(C₁-C₆), COO-alkyl(C₁-C₆), COOH, CO-alkyl(C₁-C₆), alkyl(C₀-C₆)-COOH, alkyl(C₀-C₆)-COO-alkyl(C₁-C₆), SO₂-alkyl(C₁-C₆) ;
R3 est H, alkyl(C₁-C₆), alkyl(C₀-C₆)-aryle, O-alkyl(C₁-C₆), O-alcényl(C₂-C₆), O-alcynyl(C₂-C₆), où les radicaux alkyle, aryle, alcényle et alcynyle peuvent être mono- ou polysubstitués par F, Cl ou Br ;
X est OH, O-alkyl(C₁-C₆), NH₂, NH-alkyl(C₁-C₆), N(alkyl(C₁-C₆))₂ ;
A, B, D et E sont chacun indépendamment CH ou N, et au moins un parmi les groupements A, B, D et E a pour signification N;
m vaut 1,2;
et leurs sels physiologiquement tolérés.

3. Composés de formule 1 selon la revendication 1 ou 2, **caractérisés en ce que**
R1 est H, F ;
R2 est chacun indépendamment H, F, CI, Br, alkyl(C₁-C₆), CF₃, OCF₃, O-alkyl(C₁-C₆), COO-alkyl(C₁-C₆), COOH, CO-alkyl(C₁-C₆), alkyl(C₀-C₆)-COOH, alkyl(C₀-C₆)-COO-alkyl(C₁-C₆), SO₂-alkyl(C₁-C₆) ;
R3 est H, alkyl(C₁-C₆), alkyl(C₀-C₆)-aryle, O-alkyl(C₁-C₆), O-alcényl(C₂-C₆), O-alcynyl(C₂-C₆), où les radicaux alkyle, aryle, alcényle et alcynyle peuvent être mono- ou polysubstitués par F, CI ou Br ;
X est OH, O-alkyl(C₁-C₆), NH₂, NH-alkyl(C₁-C₆), N(alkyl(C₁-C₆))₂ ;
A est N
B, D, E sont CH
m vaut 1, 2
et leurs sels physiologiquement tolérés.

4. Composés de formule I selon l'une ou plusieurs parmi les revendications 1 à 3, **caractérisés en ce que**
R1 est H, F ;
R2 est H, CI, alkyl(C₁-C₆), CF₃, COO-alkyl(C₁-C₆), COOH,
R3 est H, phényle ;
X est OH, O-alkyl(C₁-C₆), NH₂, NH-alkyl(C₁-C₆), N(alkyl(C₁-C₆))2 ;
A est N ;
B, D, E sont CH ;
m vaut 2 ;
et leurs sels physiologiquement tolérés.

5. Produit pharmaceutique comprenant un ou plusieurs composés selon l'une ou plusieurs parmi les revendications 1 à 4.

6. Produit pharmaceutique comprenant un ou plusieurs composés selon l'une ou plusieurs parmi les revendications 1 à 4, et au moins un ingrédient actif supplémentaire.

7. Produit pharmaceutique selon la revendication 6, **caractérisé en ce qu'**il comprend, comme ingrédient actif supplémentaire, un ou plusieurs parmi les anti-diabétiques, les ingrédients actifs hypoglycémiants, les inhibiteurs de la HMG-CoA réductase, les inhibiteurs de l'absorption du cholestérol, les agonistes du PPAR gamma, les agonistes du PPAR alpha, les agonistes du PPAR alpha/gamma, les fibrates, les inhibiteurs de la MTP, les inhibiteurs de l'adsorption de l'acide biliaire, les inhibiteurs de la CETP, les chélateurs de l'acide biliaire polymères, les inducteurs du récepteur de LDL, les inhibiteurs de l'ACAT, les anti-oxydants, les inhibiteurs de lipoprotéine lipase, les inhibiteurs de l'ATP citrate lyase, les inhibiteurs de la squalène synthétase, les antagonistes de la lipoprotéine (a), les inhibiteurs de la lipase, les insulines, les sulfonylurées, les biguanides, les méglitinides, les thiazolidinediones, les inhibiteurs de l'α-glucosidase, les ingrédients actifs agissant sur le canal potassique ATP-dépendant des cellules bêta, les agonistes de CART, les agonistes du NPY, les agonistes du MC4, les agonistes de l'oréxine, les agonistes du H3, les agonistes du TNF, les agonistes du CRF, les antagonistes du CRF BP, les agonistes de l'urocortine, les agonistes β3, les agonistes de la MSH (hormone de stimulation des mélanocytes), les agonistes du CCK, les inhibiteurs de recaptage de la sérotonine, les composés mixtes sérotoninergiques et noradrénergiques, les agonistes de la 5HT, les agonistes de la bombésine, les antagonistes de la galanine, les hormones de croissance, les composés de libération de l'hormone de croissance, les agonistes de la TRH, les modulateurs de la protéine 2 ou 3 de découplage, les agonistes de la leptine, les agonistes du DA (bromocriptine, Doprexine), les inhibiteurs de lipase/amylase, les modulateurs du PPAR, les modulateurs du RXR ou les agonistes du TR-β ou les amphétamines.

8. Utilisation des composés selon l'une ou plusieurs parmi les revendications 1 à 4, destinée à la production d'un médicament pour la réduction du sucre sanguin.

9. Utilisation des composés selon l'une ou plusieurs parmi les revendications 1 à 4, destinée à la production d'un médicament pour le traitement du diabète de type 2.

10. Utilisation des composés selon l'une ou plusieurs parmi les revendications 1 à 4, destinée à la production d'un médicament pour le traitement des troubles du métabolisme des glucides et des lipides.

11. Utilisation des composés selon l'une ou plusieurs parmi les revendications 1 à 4, destinée à la production d'un médicament pour le traitement des symptômes artérioscléreux.

12. Utilisation des composés selon l'une ou plusieurs parmi les revendications 1 à 4, destinée à la production d'un médicament pour le traitement de l'insulino-résistance.

13. Procédé de préparation des composés de formule 1 selon l'une ou plusieurs parmi les revendications 1 à 4, **caractérisé en ce qu'**on réagit d'urées de formule 2 ou de dérivés d'aniline de formule 3 avec des aroyl isocyanates ou avec des dérivés d'acide réactifs, tels que des chlorures d'acide ou des anhydrides, de formule 4 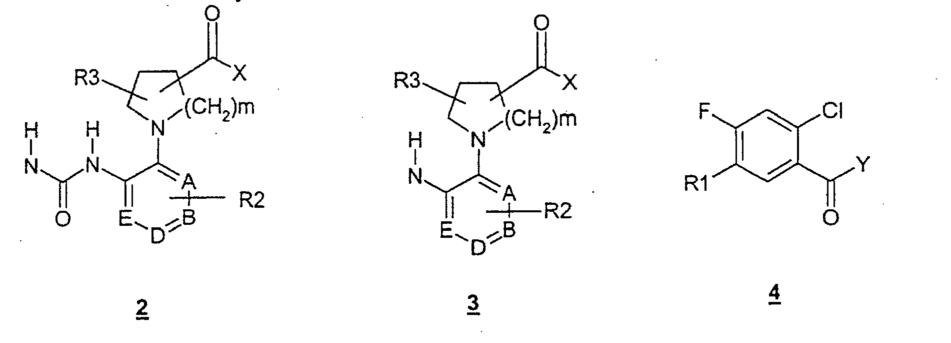 dans lesquelles R1 à R3, et A, B, D et E sont chacun tels que définis selon la revendication 1.

14. Procédé de production d'un produit pharmaceutique comprenant un ou plusieurs composés selon l'une ou plusieurs parmi les revendications 1 à 4, **caractérisé en ce qu'**on mélange de l'ingrédient actif avec un véhicule pharmaceutiquement acceptable et convertit de ce mélange en une forme convenable pour une administration.
